(19) Europäisches Patentamt
European Patent Office
Office européen des brevets

(11) Numéro de publication: **0 052 559**
**B1**

(12) # FASCICULE DE BREVET EUROPEEN

(45) Date de publication du fascicule du brevet: **24.10.84**

(51) Int. Cl.³: **C 07 C 149/16**, C 07 C 149/18, C 07 C 149/34, C 07 C 149/36

(21) Numéro de dépôt: **81401772.9**

(22) Date de dépôt: **06.11.81**

(54) **Nouveaux bromopolyfluoroalkylthioethers et leur procédé de préparation.**

(30) Priorité: **13.11.80 FR 8024117**
**25.05.81 FR 8110320**

(43) Date de publication de la demande:
**26.05.82 Bulletin 82/21**

(45) Mention de la délivrance du brevet:
**24.10.84 Bulletin 84/43**

(84) Etats contractants désignés:
**BE CH DE FR GB IT LI NL SE**

(56) Documents cités:
**TETRAHEDRON LETTERS, vol. 22, no. 4, janvier 1981, Pergamon Press Ltd., (GB), I. RICO et C. WAKSELMAN: "Synthèse de composés aromatiques comportant les groupements OCF2 Br et SCF2Br. I. Action du dibromodifluorométhane sur les thiophénate et phénate de potassium", pages 323-326**
**CHEMICAL ABSTRACTS, vol. 81, no. 17, 28 octobre 1974, ref. no. 104876d, page 474, COLUMBUS OHIO (US),**
**JOURNAL OF ORGANIC CHEMISTRY, vol. 44, no. 16, 1979, American Chemical Society, A.E. FEIRING: "Chemistry in hydrogen fluoride. 7. A novel synthesis of aryl trifluoromethyl ethers", pages 2907-2910**

(73) Titulaire: **RHONE-POULENC SPECIALITES CHIMIQUES, "Les Miroirs" 18, Avenue d'Alsace, F-92400 Courbevoie (FR)**

(72) Inventeur: **Rico, Isabelle, Villa Laurencia 19, rue de la Butte aux Cailles, F-75013 Paris (FR)**
Inventeur: **Wakselman, Claude, 18, rue du Clos d'Alençon, F 91120 Villebon-sur-Yvette (FR)**

(74) Mandataire: **Cazes, Jean-Marie et al, RHONE-POULENC RECHERCHES Service Brevets Chimie et Polymères 25, quai Paul Doumer, F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 052 559 B1

**Description**

La présente invention a pour objet de nouveaux bromopolyfluoroalkylthioéthers; elle concerne également un procédé de préparation de ces composés.

Les nouveaux composés selon l'invention ont pour formule générale:

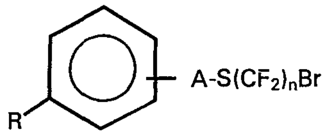 (I)

dans laquelle:

n est égal à 1 ou 2,

R représente au moins un élément choisi parmi le groupe comprenant l'hydrogène et les radicaux alkyle ayant de 1 à 12 atomes de carbone, le radical phényle, les radicaux alkyloxy ayant de 1 à 12 atomes de carbone, le radical phényloxy, les radicaux $NO_2$, CN, F, Cl, Br, I, $CF_3$, $CONR_1R_2$ et $NR_1R_2$ où $R_1$ et $R_2$ identiques ou différents, représentent chacun un hydrogène ou un radical alkyle ayant de 1 à 6 atomes de carbone, le noyau benzénique n'étant jamais perfluoré; et

A représente le radical $-CH_2-$ ou un lien valentiel.

Les composés de formule (I) ont un réel intérêt industriel dans la mesure où ils peuvent être utilisés en tant qu'intermédiaires de synthèse pour la préparation de produits ayant une activité phytosanitaire ou pharmaceutique. C'est ainsi, en particulier, que, par échange d'halogènes, ces composés donnent les perfluoroalkylthioéthers correspondants qui sont utilisés pour la préparation des produits précités.

L'invention concerne également un procédé pour la préparation des composés de formule (I) caractérisé en ce que l'onfait réagir un composé de formule:

$$Br-(CF_2)_n-X \qquad (II)$$

dans laquelle X représente le chlore ou le brome et n est égal à 1 ou 2 avec un thiolate de formule:

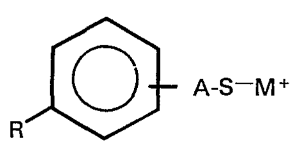 (III)

dans laquelle A et R ont la signification précédente et $M^+$ représente un cation dérivé d'un métal alcalin, dans des conditions de transfert de phase liquide-liquide en mettant en oeuvre, d'une part, un solvant aprotique non miscible à l'eau et, d'autre part, une solution aqueuse d'une base alcaline dont la concentration pondérale est d'au moins 40%, l'agent de transfert de phase étant un sel d'ammonium quaternaire.

Le thiolate de formule (III) peut être préparé soit isolement, soit in situ à partir du thiol correspondant. Dans ces cas, dans le but de simplifier la mise en oeuvre du procédé, la base alcaline utilisée pour la formation du thiolate sera celle mise en oeuvre pour le transfert de phase liquide-liquide.

Selon un mode de mise en oeuvre préférentiel du procédé selon l'invention, la solution aqueuse de base alcaline a une concentration pondérale comprise entre 40% et 60%.

Selon un autre mode de mise en oeuvre préférentiel de l'invention, on utilise comme solution aqueuse de base alcaline, une solution de potasse, la réaction faisant alors intervenir le thiolate de potassium. La demanderesse a en effet constaté que c'est dans ce cas que la quantité du produit secondaire principal à savoir le composé:

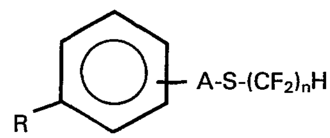

est la plus faibe et, donc, que le rendement en produit recherché est le meilleur.

Il est à souligner que l'utilisation d'autres bases alcalines comme la soude par exemple et d'autres thiolates alcalins comme le thiolate de sodium par exemple n'est pas exclue du cadre de la présente invention. N'est non plus pas exclue, bienque d'une mise en oeuvre inutilement plus compliquée, l'utilisation simultanée d'un thiolate dont le cation alcalin ne serait pas identique à celui de la base alcaline.

Le solvant aprotique non miscible à l'eau est choisi de préférence parmi le groupe comprenant le benzène, le toluène et les xylènes.

On préfère tout particulièrement mettre en oeuvre le benzène ou le toluène.

Le sel d'ammonium quaternaire est de préférence choisi parmi les halogénures de tétraalkyl ammonium.

On peut citer comme exemples de sels d'ammonium quaternaire convenant particulièrement bien pour le procédé de l'invention, les chlorures et bromures de tétrabutylammonium et de triéthyl benzyl ammonium.

On utilise le thiolate de formule (III) et le composé de formule (II) en quantités telles que le nombre de moles de II par mole de III est compris de préférence entre 1 et 3 et, encore plus préférentiellement, entre environ 1 et environ 2.

Le nombre de moles d'agent de transfert de phase per mole de thiolate de solvant organique est compris de préférence entre 0,01 et 0,1. Encore plus préférentiellement, il est compris entre 0,03 et 0,06.

On utilise de préférence entre 1 et 5 litres de solvant organique par mole de thiolate de formule (III). Encore plus préférentiellement, on en utilise entre 1,5 et 3 litres.

Pour une bonne mise en oeuvre de l'invention, on préfère utiliser des volumes sensiblement égaux de solvant organique et de solution aqueuse de base alcaline.

Le procédé selon l'invention peut être mis en oeuvre à une température comprise entre 0°C et 100°C. On préfère opérer à la température ambiante.

On opère de préférence à la pression atmosphérique bien que des pressions supérieures ou inférieures à la pression atmosphérique ne soient pas exclues du domaine de l'invention.

Une manière pratique de mettre en oeuvre le procédé selon l'invention, quand dans le composé (II) utilisé X = Br, consiste à mélanger sous agitation la solution aqueuse de base alcaline, le solvant organique et l'agent de transfert de phase. On ajoute ensuite simultanément le thiol et le composé II de façon à transférer progressivement le thiolate qui se forme dans la phase aqueuse (quand le thiol est solide, on le dissout préalablement dans une faible quantité de solvant organique).

On laisse le mélange résultant sous agitation pendant le temps nécessaire à la réaction. Après addition d'eau, la phase organique est décantée puis lavée à l'eau. On évapore le solvant organique. L'huile obtenue est purifiée par entraînement à la vapeur d'eau. On sèche ensuite sur carbonate de sodium par exemple. Le produit de la réaction est ensuite séparé soit par distillation à la bande tournante quand il est liquide soit par chromatographie sur plaques quand il est solide.

L'ajout simultané du composé II et du thiol est préférable pour limiter la réaction de dimérisation du thiol en disulfure.

Cet ajout simultané n'est pas nécessaire quand dans le composé (II) n = 1 et X = Cl. Dans ce cas, on condense au préalable $BrCF_2Cl$ qui est gazeux et on l'ajoute en totalité au mélange contenant la totalité du thiolate.

Les temps de réaction sont compris généralement entre 2 et 6 heures environ.

Les composés de formule II et III sont préparés selon des techniques bien connues de l'homme de l'art.

L'invention va maintenant être plus complètement décrite dans les exemples qui vont suivre. Ces exemples ne sauraient être interprétés comme limitant de façon quelconque l'invention définie par les revendications.

*Exemple 1*

Préparation de bromodifluorométhylthio,méthoxy-4 benzène

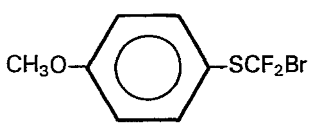 par action de $CF_2Br_2$ sur

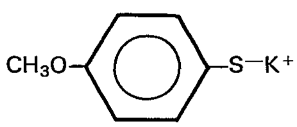

Dans un ballon surmonté d'un réfrigérant à carboglace, on mélange sous agitation à 20°C, 200 ml d'une solution aqueuse de potasse à 50% en poids, 200 ml de benzène et 0,5 g (0,002 mole) de chlorure de triéthylbenzylammonium $C_6H_5CH_2\overset{+}{N}(Et)_3Cl^-$. On ajoute ensuite simultanément 14 g (0,1 mole) de:

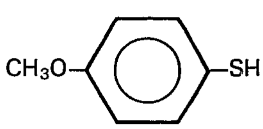

et 42 g (0,2 mole) de $CF_2Br_2$.

La température s'élève jusqu'à 30°C. On laisse ensuite sous agitation le mélange pendant quatre heures. Après addition de 200 ml d'eau, la phase organique est décantée et lavée à l'eau trois fois (3 × 70 ml). Le benzène est évaporé. L'huile obtenue est ensuite soumise à un antraînement à la vapeur d'eau, puis séchée sur carbonate de sodium. On sépare par distillation à la bande tournante 16,1 g d'un produit dont le point d'ébullition est $EB_{8mmHg} = 115°C$ et dont l'analyse RMN du proton et du fluor révèlent la formule:

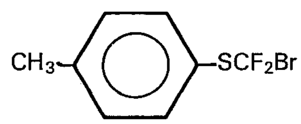

*Exemple 2*

Préparation du bromodifluorométhylthio,méthoxy-4 benzène

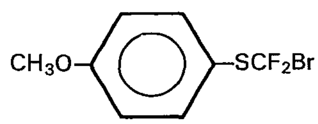 par action de $CF_2BrCl$

sur 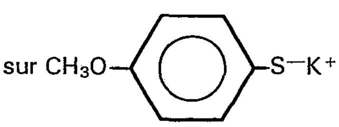

Dans un ballon surmonté d'un réfrigérant à carboglace, on mélange sous agitation à 20°C, 200 ml d'une solution aqueuse de potasse à 50% en poids, 200 ml de benzène et 0,5 g (0,002 mole) de chlorure de triéthylbenzylammonium $C_6H_5CH_2\overset{+}{N}(Et)_3Cl^-$. On ajoute successivement 14 g (0,1 mole) de:

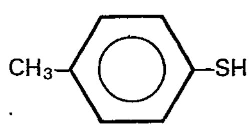

puis 33 g (0,2 mole) de $CF_2BrCl$ préalablement condensés dans un bain carboglace-acétone. La température s'élève jusqu'à 25°C. On opère ensuite comme dans l'exemple 1. On sépare finalement par distillation à la bande tournante 13,1 g de:

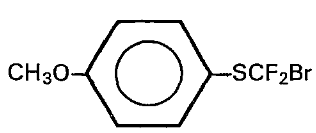

défini dans l'exemple 1.

*Exemple 3*

Préparation du bromodifluorométhylthiobenzène

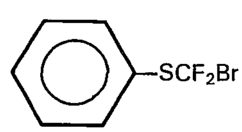 par action de $CF_2Br_2$ sur

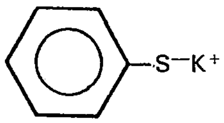

On opère comme dans l'exemple 1 avec 11 g (0,1 mole) de:

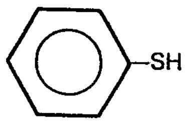

Par distillation à la bande tournante, on sépare 11,6 g d'un produit dont le point d'ébullition est $Eb_{34mmHg}$ = 97°C et dont l'analyse RMN du proton et du fluor révèlent la formule:

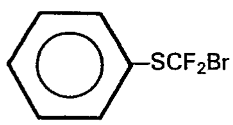

*Exemple 4*

Préparation du bromodifluorométhylthiobenzène

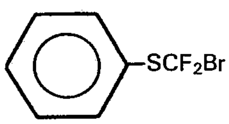 par action de $CF_2BrCl$ sur

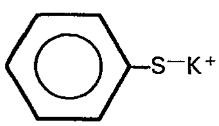

On opère comme dans l'exemple 2 avec 11 g (0,1 mole) de:

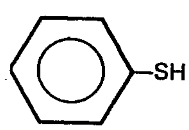

Par distillation à la bande tournante, on sépare 10,8 g de:

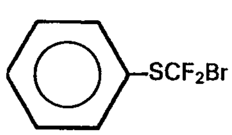

défini dans l'exemple 3.

*Exemple 5*

Préparation du bromodifluorométhylthio,bromo-4 benzène

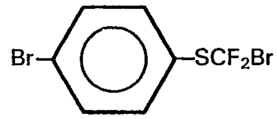 par action de $CF_2Br_2$ sur

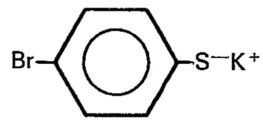

On opère dans l'exemple 1 avec 18,9 g (0,1 mole) de:

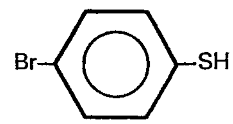

Par distillation à la bande tournante, on sépare 4,7 g d'un produit dont le point d'ébullition est $Eb_{32mmHg}$ = 125°C et dont l'analyse RMN du proton et du fluor révèlent la formule:

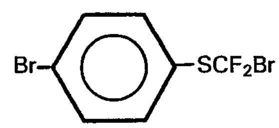

*Exemple 6*

Préparation du bromodifluorométhylthio,bromo-4 benzène

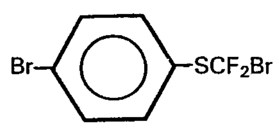 par action de $CF_2BrCl$ sur

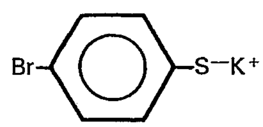

On opère comme dans l'exemple 2 avec 18,9 g (0,1 mole) de:

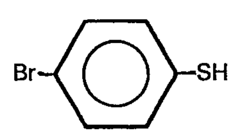

Par distillation à la bande tournante, on sépare 2,9 g de:

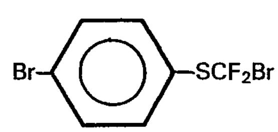

défini dans l'exemple 5.

*Exemple 7*

Préparation du bromodifluorométhylthio,nitro-4 benzène

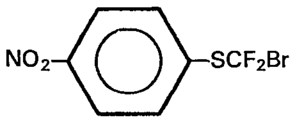 par action de $CF_2Br_2$ sur

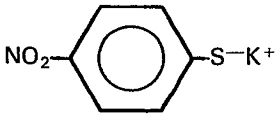

On opère comme dans l'exemple 1 avec 15,5 g (0,1 mole) de:

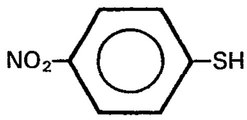

Par chromatographie sur plaques de silice (Eluat benzène), on sépare 0,71 g d'un produit dont le point de fusion est P.F. = 55°C et dont l'analyse RMN du proton et du fluor révèlent la formule:

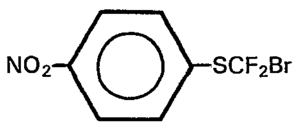

*Exemple 8*

Préparation du bromodifluorométhylthio,nitro-4 benzène

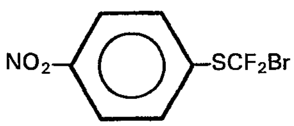 par action de $CF_2BrCl$ sur

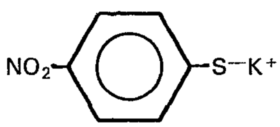

on opère comme dans l'exemple 2 avec 15,5 g (0,1 mole) de:

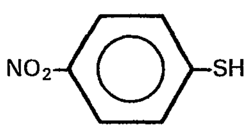

Par chromatographie sur plaque de silice (Eluat benzène), on sépare 0,42 g de:

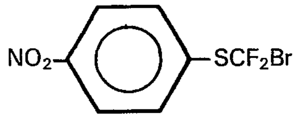

défini dans l'exemple 7.

*Exemple 9*

Préparation du bromodifluorométhyl,benzylthio-éther

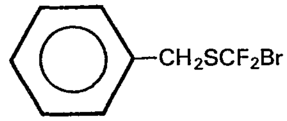 par action de $CF_2Br_2$ sur

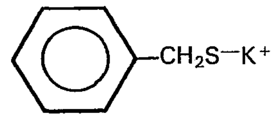

On opère comme dans l'exemple 1 avec 12,4 g (0,1 mole) de:

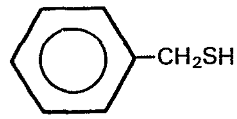

Par distillation à la bande tournante, on sépare 6,3 g d'un produit dont le point d'ébullition est $Eb_{34mmHg}$ = 120°C et dont l'analyse RMN du proton et du fluor révèlent la formule:

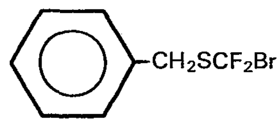

*Exemple 10*

Préparation du bromodifluorométhyl,benzylthio-éther

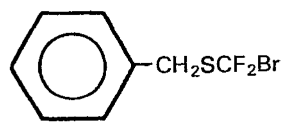 par action de $CF_2BrCl$ sur

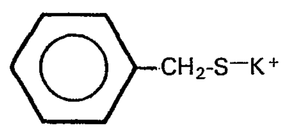

On opère comme dans l'exemple 2 avec 12,4 g (0,1 mole) de:

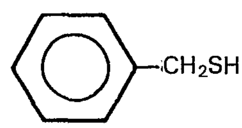

Par distillation à la bande tournante, on sépare 5,7 g de:

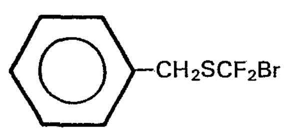

défini dans l'exemple 9.

*Exemple 11*

Préparation du β-bromotétrafluoroéthylthio,méthyl-4 benzène

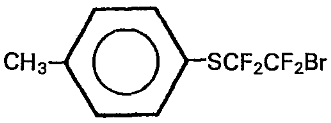 par action de

BrCF$_2$CF$_2$Br sur 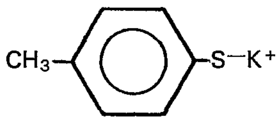

Dans un ballon surmonté d'un réfrigérant à carboglace, on mélange sous agitation à 20°C, 200 ml d'une solution aqueuse de potasse à 50% en poids, 200 ml de benzène et 0,5 g (0,002 mole) de chlorure de triéthylbenzylammonium $C_6H_5CH_2\overset{+}{N}(Et)_3Cl^-$. On ajoute ensuite simultanément 12,4 g (0,1 mole) de

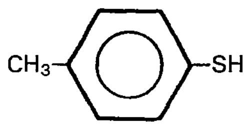

et 39 g (0,15 mole) de BrCF$_2$CF$_2$Br. La température s'élève de 20 à 25°C. On laisse ensuite sous agitation le mélange pendant 6 heures. Après addition de 200 ml d'eau, la phase organique est décantée et lavée à l'eau trois fois (3 × 70 ml). La benzène est évaporé. L'huile obtenue est ensuite soumise à un entraînement à la vapeur d'eau puis séchée sur carbonate de sodium. On sépare par distillatin à la bande tournante 7,5 g d'un produit dont le point d'ébullition est $Eb_{40mmHg}$ = 117°C et dont l'analyse RMN du proton et du fluor révèlent la formule:

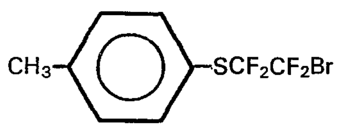

*Exemple 12*

Préparation du β-bromotétrafluoroéthylthiobenzène

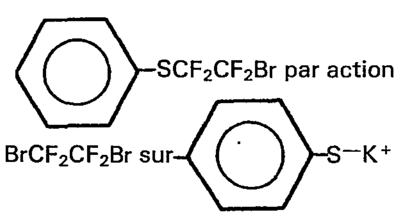 par action de

BrCF$_2$CF$_2$Br sur

On opère comme dans l'exemple 11 avec 11 g (0,1 mole) de

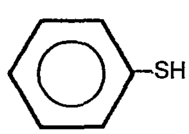

Par distillation à la bande tournante on sépare 4 g d'un produit dont le point d'ébullition est $Eb_{40mmHg}$ = 99°C et dont l'analyse RMN du proton et du fluor révèlent la formule:

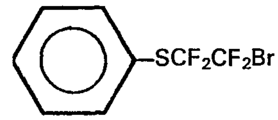

*Exemple 13*

Préparation du β-bromotétrafluoroéthylthio,chloro-4 benzène

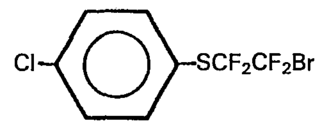

On opère comme dans l'exemple 11 avec 14,4 g (0,1 mole) de:

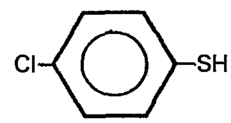

Par distillation à la bande tournante on sépare 2,3 g d'un produit dont le point d'ébullition est $Eb_{25mmHg}$ = 115°C et dont l'analyse RMN du proton et du fluor révèlent la formule:

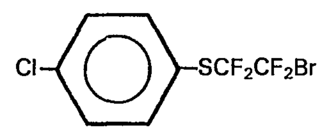

*Exemple 14*

Préparation du β-bromotétrafluoroéthylthio,nitro-4 benzène

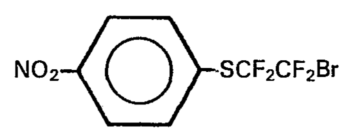

On opère comme dans l'exemple 11 avec 15,5 g (0,1 mole) de:

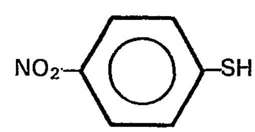

Par chromatographie sur plaques de silice (Eluant benzène), on sépare 0,5 g d'un produit dont le point de fusion est P.F. = 75°C et dont l'analyse RMN du proton et du fluor révèlent la formule:

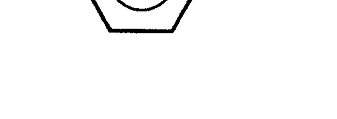

**Revendications**

1. Bromopolyfluoroalkylthioéthers de formule:

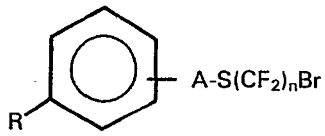

(I)

dans laquelle:

n est égal à 1 ou 2,

R représente au moins un élément choisi parmi le groupe comprenant l'hydrogène et les radicaux alkyle ayant de 1 à 12 atomes de carbone, le radical phényle, les radicaux alkyloxy ayant de 1 à 12 atomes de carbone, le radical phényloxy, les radicaux $NO_2$, CN, F, Cl, Br, I, $CF_3$, $CONR_1R_2$ et $NR_1R_2$ où $R_1$ et $R_2$ identiques ou différents, représentent chacun un hydrogène ou un radical alkyle ayant de 1 à 6 atomes de carbone, le noyau benzénique n'étant jamais perfluoré; et

A représente le radical $-CH_2-$ ou un lien valentiel.

2. Bromopolyfluoroalkylthioéther selon la revendication 1 répondant à la formule:

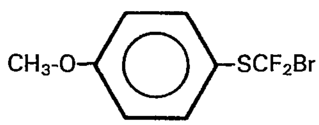

3. Bromopolyfluoroalkylthioéther selon la revendication 1 répondant à la formule:

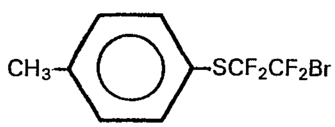

4. Bromopolyfluoroalkylthioéther selon la revendication 1 répondant à la formule:

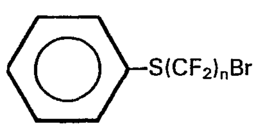

5. Bromopolyfluoroalkylthioéther selon la revendication 1 répondant à la formule:

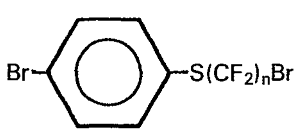

6. Bromopolyfluoroalkylthioéther selon la revendication 1 répondant à la formule:

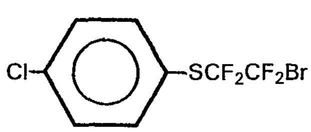

7. Bromopolyfluoroalkylthioéther selon la revendication 1 répondant à la formule:

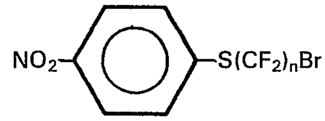

8. Bromopolyfluoroalkylthioéther selon la revendication 1 répondant à la formule:

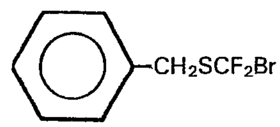

9. Procédé pour la préparation des composés selon l'une quelconque des revendications précédentes, caractérisé en ce que l'on fait réagir un composé de formule:

$$Br(CF_2)_nX \qquad (II)$$

dans laquelle X représente le chlore ou le brome et n est égal à 1 ou 2 avec un thiolate de formule:

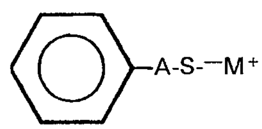

dans laquelle A et R sont tels que définis dans la revendication 1 et $M^+$ représente un cation dérivé d'un métal alcalin, dans des conditions de transfert de phase liquide-liquide en mettant en oeuvre, d'une part, un solvant aprotique non miscible à l'eau et, d'autre part, une solution aqueuse d'une base alcaline dont la concentration pondérale est d'au moins 40%, l'agent de transfert de phase étant un sel d'ammonium quaternaire.

10. Procédé selon la revendication 9, caractérisé en ce que la concentration pondérale de la solution aqueuse de base alcaline est comprise entre 40% et 60%.

11. Procédé selon la revendication 9, caractérisé en ce que le thiolate de formule III est un thiolate de potassium.

12. Procédé selon la revendication 9, caractérisé en ce que la solution aqueuse de la base alcaline mise en oeuvre est une solution aqueuse de potasse.

13. Procédé selon la revendication 9, caractérisé en ce que le solvant non miscible à l'eau est choisi parmi le groupe comprenant le benzène, le toluène et les xylènes.

14. Procédé selon la revendication 9, caractérisé en ce que le solvant est le benzène ou le toluène.

15. Procédé selon la revendication 9, caractérisé en ce que le sel d'ammonium quaternaire est un halogénure de tétraalkylammonium.

16. Procédé selon la revendication 13, caractérisé en ce que le sel d'ammonium quaternaire est le chlorure de triéthylbenzylammonium.

17. Procédé selon la revendication 9, caractérisé en ce que l'on utilise le thiolate de formule III et le composé de formule II en quantités telles que le nombre de moles de II par mole de III est compris entre 1 et 3.

18. Procédé selon la revendication 9, caractérisé en ce que le nombre de moles d'agent de transfert de phase par mole de thiolate de formule III est compris entre 0,01 et 0,1.

19. Procédé selon la revendication 9, caractérisé en ce qu'on utilise entre 1 et 5 litres de solvant organique par mole de thiolate (III).

20. Procédé selon l'une quelconque des revendications précédentes, caractérisé en ce qu'on opère à une température comprise entre 0 et 100°C sous pression atmosphérique.

**Patentansprüche**

1. Bromopolyfluoralkylthioether der Formel:

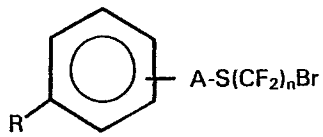

(I)

in der

n = 1 oder 2 ist,

R mindestens ein Substituent ist aus der Gruppe von Wasserstoff, den Alkylresten mit 1 bis 12 Kohlenstoffatomen, dem Phenylrest, den Alkoxyresten mit 1 bis 12 Kohlenstoffatomen, dem Phenyloxyrest, den Resten $-NO_2$, -CN, -F, -Cl, -Br, -I, $-CF_3$, $-CONR_1R_2$ und $NR_11R_2$ mit identischen oder verschiedenen $R_1$ und $R_2$, die ein Wasserstoffatom oder einen Alkylrest mit 1 bis 6 Kohlenstoffatomen bedeuten, wobei der Benzolkern nie perfluoriert ist; und

A die $-CH_2$-Gruppe oder eine Valenzbindung bedeutet.

2. Brompolyfluoralkylthioether nach Anspruch 1 mit der Formel:

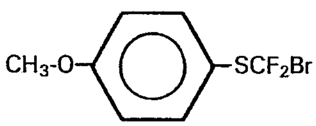

3. Brompolyfluoralkylthioether nach Anspruch 1 mit der Formel:

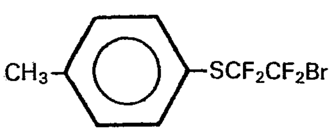

4. Brompolyfluoralkylthioether nach Anspruch 1 mit der Formel:

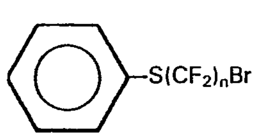

5. Brompolyfluoralkylthioether nach Anspruch 1 mit der Formel:

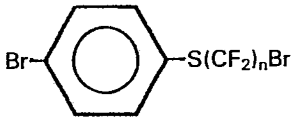

6. Brompolyfluoralkylthioether nach Anspruch 1 mit der Formel:

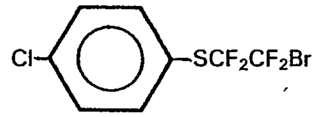

7. Brompolyfluoralkylthioether nach Anspruch 1 mit der Formel:

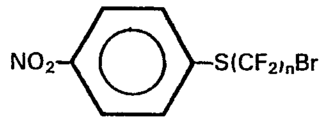

8. Brompolyfluoralkylthioether nach Anspruch 1 mit der Formel:

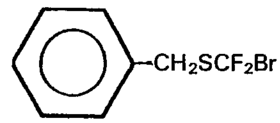

9. Verfahren zur Herstellung der Verbindungen nach einem oder mehreren der vorangehenden Ansprüche, dadurch gekennzeichnet, dass man eine Verbindung der Formel

$$Br(CF_2)_nX \qquad (II)$$

in der X Chlor oder Brom darstellt und n = 1 oder 2 ist, mit einem Thiolat der Formel:

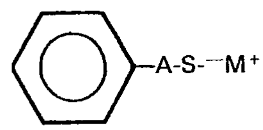

(III)

in der A und R so wie im Anspruch 1 definiert sind und $M^+$ ein Alkalimetallkation ist, unter Flüssig-Flüssig-Phasentransfert-Bedingungen umsetzt, wobei man einerseits ein aprotisches, mit Wasser nicht mischbares Lösungsmittel und andererseits eine wässrige Alkalilauge einer Konzentration von mindestens 40 Gew.-% einsetzt, und das Phasenübertragungsmittel ein quartäres Ammoniumsalz ist.

10. Verfahren nach Anspruch 9, dadurch gekennzeichnet, dass die Konzentration der wässrigen Alkalibase 40 bis 60 Gew.-% beträgt.

11. Verfahren nach Anspruch 9, dadurch gekennzeichnet, dass das Thiolat der Formel III ein Kaliumthiolat ist.

12. Verfahren nach Anspruch 9, dadurch gekennzeichnet, dass die eingesetzte wässrige Alkalibase eine wässrige Lösung von Kaliumhydroxid ist.

13. Verfahren nach Anspruch 9, dadurch gekennzeichnet, dass das mit Wasser nicht mischbare Lösungsmittel aus der Gruppe von Benzol, Toluol und der Xylole gewählt wird.

14. Verfahren nach Anspruch 9, dadurch gekennzeichnet, dass das Lösungsmittel Benzol oder Toluol ist.

15. Verfahren nach Anspruch 9, dadurch gekennzeichnet, dass das quartäre Ammoniumsalz ein Tetraalkylammoniumhalogenid ist.

16. Verfahren nach Anspruch 13, dadurch gekennzeichnet, dass das quartäre Ammoniumsalz Triethylbenzylammoniumchlorid ist.

17. Verfahren nach Anspruch 9, dadurch gekennzeichnet, dass man das Thiolat der Formel III und die Verbindung der Formel II in solchen Mengen verwendet, dass das Molverhältnis zwischen der Verbindung II und III zwischen 1 und 3 beträgt.

18. Verfahren nach Anspruch 9, dadurch gekennzeichnet, dass das Molverhältnis zwischen dem Phasentransfermittel und dem Thiolat der Formel III zwischen 0,01 und 0,1 beträgt.

19. Verfahren nach Anspruch 9, dadurch gekennzeichnet, dass man zwischen 1 und 5 Liter organisches Lösungsmittel pro Mol Thiolat (III) verwendet.

20. Verfahren nach einem oder mehreren der vorangehenden Ansprüche, dadurch gekennzeichnet, dass man bei einer Temperatur zwischen 0 und 100°C unter Atmosphärendruck arbeitet.

**Claims**

1. Bromopolyfluoroalkyl thioethers of the formula

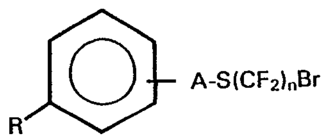

(I)

in which:

n is 1 or 2,

R représents at least one element chosen from among the group comprising hydrogen and alkyl radicals having from 1 to 12 carbon atoms, the phenyl radical, alkoxy radicals having from 1 to 12 carbon atoms, the phenoxy radical and the radicals $NO_2$, CN, F, Cl, Br, I, $CF_3$, $CONR_1R_2$ and $NR_1R_2$, where $R_1$ and $R_2$, which may be identical or different, each represent hydrogen or an alkyl radical having from 1 to 6 carbon atoms, but the benzene ring is never perfluorinated, and

A represents the $-CH_2-$ radical or a valency bond.

2. Bromopolyfluoroalkyl thioether according to Claim 1, corresponding to the formula:

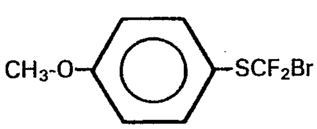

3. Bromopolyfluoroalkyl thioether according to Claim 1, corresponding to the formula:

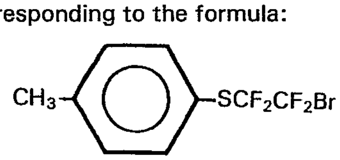

4. Bromopolyfluoroalkyl thioether according to Claim 1, corresponding to the formula:

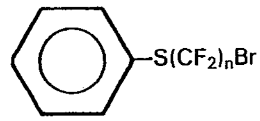

5. Bromopolyfluoroalkyl thioether according to Claim 1, corresponding to the formula:

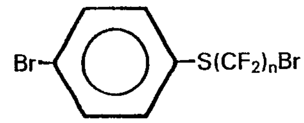

6. Bromopolyfluoroalkyl thioether according to Claim 1, corresponding to the formula:

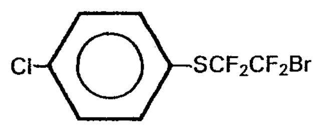

7. Bromopolyfluoroalkyl thioether according to Claim 1, corresponding to the formula:

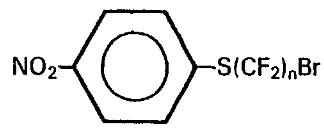

8. Bromopolyfluoroalkyl thioether according to Claim 1, corresponding to the formula:

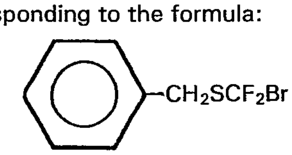

9. Process for the preparation of the compounds according to any one of the preceding claims, characterised in that a compound of the formula:

$$Br(CF_2)_nX \quad (II)$$

in which X représents chlorine or bromine and n is 1 or 2 is reacted with a thiolate of the formula:

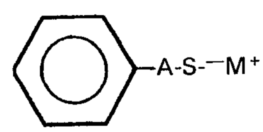

in which A and R are as defined in claim 1 and $M^+$ represents a cation derived from an alkali metal, under liquid-liquid phase transfer conditions, employing, on the one hand, a water-immiscible aprotic solvent and, on the other hand, an aqueous solution of an alkali metal base, the weight concentration of which is at least 40%, the phase transfer agent being a quaternary ammonium salt.

10. Process according to Claim 9, characterised in that the weight concentration of the aqueous solution of an alkali metal base is between 40% and 60%.

11. Process according to Claim 9, characterised in that the thiolate of formula III is a potassium thiolate.

12. Process according to Claim 9, characterised in that the aqueous solution of the alkali metal base employed is an aqueous potassium hydroxide solution.

13. Process according to Claim 9, characterised in that the water-immiscible solvent is chosen from the group comprising benzene, toluene and the xylenes.

14. Process according to Claim 9, characterised in that the solvent is benzene or toluene.

15. Process according to Claim 9, characterised in that the quaternary ammonium salt is a tetraalkylammonium halide.

16. Process according to Claim 13, characterised in that the quaternary ammonium salt is triethylbenzylammonium chloride.

17. Process according to Claim 9, characterised in that the thiolate of the formula III and the compound of the formula II are employed in such amounts that the number of moles of II per mole of III is between 1 and 3.

18. Process according to Claim 9, characterised in that the number of moles of phase transfer agent per mole of thiolate of formula III is between 0.01 and 0.1.

19. Process according to Claim 9, characterised in that between 1 and 5 litres of organic solvent are used per mole of thiolate (III).

20. Process according to any one of the preceding claims, characterised in that it is carried out at a temperature of between 0 and 100°C, under atmospheric pressure.